# EUROPEAN PATENT APPLICATION

(11) **EP 4 014 960 A1**
(43) Date of publication of application: **22.06.2022**
(21) Application number: 20851828.2
(22) Date of filing: 17.08.2020
(51) Int. Cl.: A61K 8/92, A61Q 19/08, A61Q 17/04, A61K 35/35, A61P 17/00, A61P 17/06, A61P 17/02, A61P 3/10, C11B 1/00, C11B 1/02, C11B 3/00, C12N 5/071

(54) **FAT EXTRACT WITHOUT ADDED INGREDIENTS, PREPARATION METHOD THEREFOR AND USE THEREOF**

(30) Priority: 15.08.2019 CN 201910755679
(71) Applicant: Shanghai Seme Cell Technology Co., Ltd, Shanghai 200241 (CN)
(72) Inventor: LI, Wei, Shanghai 200241 (CN); ZHANG, Wenjie, Shanghai 200241 (CN); DENG, Mingwu, Shanghai 200241 (CN); XU, Yuda, Shanghai 200241 (CN); YU, Ziyou, Shanghai 200241 (CN); WANG, Xiangsheng, Shanghai 200241 (CN); CAI, Yizuo, Shanghai 200241 (CN); ZHENG, Hongjie, Shanghai 200241 (CN)
(74) Representative: Mewburn Ellis LLP
(86) International application number: PCT/CN2020/109651
(87) International publication number: WO 2021/027971

(57) **Abstract**

The present invention provides a fat extract without added ingredients, a preparation method therefor and a use thereof. In particular, the present invention provides a use of a fat extract without added ingredients in the preparation of a composition or product for one or more uses selected from the group consisting of: (a) promoting proliferation of fibroblasts; (b) promoting anti-aging of fibroblasts; and (c) promoting production of type I collagen in fibroblasts. The present invention further provides a preparation method for the fat extract without added ingredients, a pharmaceutical or cosmetic composition containing the fat extract without added ingredients, and a method for culturing fibroblasts in vitro. The fat extract without added ingredients provided in the present invention can effectively and cooperatively inhibit skin aging, prevent fibroblast apoptosis, promote fibroblast proliferation and collagen synthesis, and promote skin rejuvenation.

## Description

### TECHNICAL FIELD

The invention belongs to the field of biotechnology, in particular, relates to a fat extract without added ingredients, preparation method therefor and use thereof.

### BACKGROUND TECHNIQUE

Skin is the first protective barrier of the body and plays an important role in resisting external aggression and maintaining the stability of internal environment. Skin aging can be divided into endogenous aging and exogenous aging: the programmed aging process controlled by genetic factors is endogenous aging or natural aging, which develops with time and is irresistible; aging caused by environmental factors is exogenous aging, the most important of which is skin aging caused by repeated exposure to ultraviolet rays, that is, photoaging.

The essence of ultraviolet damage to cells is that the photon energy of ultraviolet rays is absorbed by the atoms or molecules of cells, causing the electronic energy level of atoms to change, generating a large number of secondary electrons and free radicals, which in turn lead to a variety of damage events in the cells. Nucleic acids and proteins have absorption peaks at wavelengths of 260 nm and 278 nm, respectively, which are within the UV wavelength range. The photon energy of UVB can be directly absorbed by nucleic acids and proteins, resulting in cell damage; UVA mediates energy transfer through photosensitizers in cells to form free radicals, causing cell damage. The accumulation of cellular damage leads to apparent skin aging.

Although some anti-aging technologies and products have been developed in the art, their anti-aging effects are still unsatisfactory. Therefore, there is an urgent need in the art to develop new products that can effectively and safely enhance skin anti-aging.

### SUMMARY OF THE INVENTION

The object of the present invention is to provide a fat extract without added ingredients and use thereof, and the fat extract can effectively and safely enhance the vitality of fibroblasts and have significant effects in anti-aging.

In the first aspect of the present invention, it provides a use of a fat extract without added ingredients, for the manufacture of a composition or product, the composition or product is used for one or more uses selected from the group consisting of (a) promoting proliferation of fibroblasts; (b) promoting anti-aging of fibroblasts; (c) promoting production of type I collagen in fibroblasts.

In another preferred embodiment, the fibroblasts comprise skin fibroblasts.

In another preferred embodiment, the aging comprises cell aging caused by ultraviolet irradiation.

In another preferred embodiment, the ultraviolet ray comprises UVB, UVA, and a combination thereof.

In another preferred embodiment, the composition or product is also used for preventing and/or repairing skin damage caused by damage or decreased vitality of fibroblasts.

In another preferred embodiment, the skin damage comprises skin barrier damage.

In another preferred embodiment, the skin damage comprises photoaging, polymorphic light eruption.

In another preferred embodiment, the composition is also used for preventing and/or treating skin disease.

In another preferred embodiment, the skin disease is selected from the group consisting of lupus erythematosus, vitiligo, psoriasis, chloasma, diabetic skin ulcer, and allergic purpura.

In another preferred embodiment, the composition comprises a pharmaceutical composition, a cosmetic composition.

In another preferred embodiment, the product is selected from the group consisting of medicine, cosmetic, and health care product.

In another preferred embodiment, the pharmaceutical composition comprises injection, injection, and external preparation.

In another preferred embodiment, the pharmaceutical composition is administered by external administration, topical administration, or subcutaneous injection.

In another preferred embodiment, the cosmetic composition is administered externally.

In another preferred embodiment, the fat extract contains no cell and no lipid droplet.

In another preferred embodiment, the lipid droplets are oil droplets released after fat cells are disrupted.

In another preferred embodiment, the expression of "contain no lipid droplet" means that in the fat extract, the volume of oil droplets accounts for less than 1% of the total liquid, preferably less than 0.5%, more preferably less than 0.1% of the total liquid.

In another preferred embodiment, the cells are selected from the group consisting of endothelial cells, adipose stem cells, macrophages, and stromal cells.

In another preferred embodiment, the expression of "contain no cell "refers to the average number of cells in 1 ml of the fat extract is ≤ 1, preferably ≤ 0.5, more preferably ≤ 0.1, or 0.

In another preferred embodiment, the fat extract is not SVF.

In another preferred embodiment, the fat extract is a naturally-obtained nano-fat extract without added ingredients.

In another preferred embodiment, the expression of "without added ingredients" refers to no solution, solvent, small molecule, chemical, and biological additive are added during the preparation of the fat extract except rinsing step.

In another preferred embodiment, the fat extract is prepared by centrifuging the fat tissue after emulsification.

In another preferred embodiment, the fat extract contains, but is not limited to, one or more components selected from the group consisting of growth factors IGF-1, BDNF, GDNF, TGF-β, HGF, bFGF, VEGF, PDGF, EGF, NT-3, GH, G-CSF, and combinations thereof.

In another preferred embodiment, the fat extract without added ingredients contains one or more components selected from the group consisting of IGF-1, BDNF, GDNF, TGF-β, HGF, bFGF, VEGF, TGF-β1, HGF, PDGF, EGF, NT-3, GH, G-CSF, and combinations thereof.

In another preferred embodiment, the fat extract without added ingredients contains, but is not limited to, one or more components selected from the group consisting of IGF-1, BDNF, GDNF, bFGF, VEGF, TGF-β1, HGF, PDGF, and combinations thereof.

In another preferred embodiment, in the fat extract without added ingredients, the concentration of IGF-1 is 5000-30000 pg/ml, preferably 6000-20000 pg/ml, more preferably 7000-15000 pg/ml, more preferably 8000-12000 pg/ml, more preferably 9000-11000 pg/ml, more preferably 9500-10500 pg/ml.

In another preferred embodiment, in the fat extract without added ingredients, the concentration of BDNF is 800-5000 pg/ml, preferably 1000-4000 pg/ml, more preferably 1200-2500 pg/ml pg/ml, more preferably 1400-2000 pg/ml, more preferably 1600-2000 pg/ml, more preferably 1700-1850 pg/ml.

In another preferred embodiment, in the fat extract without added ingredients, the concentration of GDNF is 800-5000 pg/ml, preferably 1000-4000 pg/ml, more preferably 1200-2500 pg/ml pg/ml, more preferably 1400-2000 pg/ml, more preferably 1600-2000 pg/ml, more preferably 1700-1900 pg/ml.

In another preferred embodiment, in the fat extract without added ingredients, the concentration of bFGF is 50-600 pg/ml, preferably 100-500 pg/ml, more preferably 120-400 pg/ml, more preferably 150-300 pg/ml, more preferably 200-280 pg/ml, more preferably 220-260 pg/ml.

In another preferred embodiment, in the fat extract without added ingredients, the concentration of the VEGF is 50-500 pg/ml, preferably 100-400 pg/ml, more preferably 120-300 pg/ml, more preferably 150-250 pg/ml, more preferably 170-230 pg/ml, more preferably 190-210 pg/ml.

In another preferred embodiment, in the fat extract without added ingredients, the concentration of TGF-β1 is 200-3000 pg/ml, preferably 400-2000 pg/ml, more preferably 600-1500 pg/ml, more preferably 800-1200 pg/ml, more preferably 800-1100 pg/ml, more preferably 900-1000 pg/ml.

In another preferred embodiment, in the fat extract without added ingredients, the concentration of the HGF is 200-3000 pg/ml, preferably 400-2000 pg/ml, more preferably 600-1500 pg/ml, more preferably 600-1200 pg/ml, more preferably 800-1000 pg/ml, more preferably 850-950 pg/ml.

In another preferred embodiment, in the fat extract without added ingredients, the concentration of PDGF is 50-600 pg/ml, preferably 80-400 pg/ml, more preferably 100-300 pg/ml, more preferably 140-220 pg/ml, more preferably 160-200 pg/ml, more preferably 170-190 pg/ml.

In another preferred embodiment, the weight ratio of IGF-1 to VEGF is 20-100:1, preferably 30-70:1, more preferably 40-60:1, and most preferably 45-55:1.

In another preferred embodiment, the weight ratio of BDNF to VEGF is 2-20:1, preferably 4-15:1, more preferably 6-12:1, and most preferably 8-9.5:1.

In another preferred embodiment, the weight ratio of GDNF to VEGF is 2-20:1, preferably 4-15:1, more preferably 6-12:1, and most preferably 8.5-9.5:1.

In another preferred embodiment, the weight ratio of bFGF to VEGF is 0.2-8:1, preferably 0.5-5:1, more preferably 0.6-2:1, more preferably 0.8-1.6:1, and most preferably 1-1.5:1.

In another preferred embodiment, the weight ratio of TGF-β1 to VEGF is 1-20:1, preferably 1-15:1, more preferably 1-10:1, more preferably 2-8:1, more preferably 4-6:1.

In another preferred embodiment, the weight ratio of HGF to VEGF is 1-20:1, preferably 1-15:1, more preferably 1-10:1, more preferably 2-8:1, more preferably 4-5.5:1.

In another preferred embodiment, the weight ratio of PDGF to VEGF is 0.1-3:1, preferably 0.2-2:1, more preferably 0.4-1.5:1, and most preferably 0.7-1.2:1.

In another preferred embodiment, the fat extract without added ingredients is liquid.

In another preferred embodiment, the fat extract without added ingredients is prepared by the method described in the second aspect of the present invention.

In the second aspect of the present invention, it provides a method for preparing a fat extract without added ingredients, comprising the following steps:
(1) providing an fat tissue raw material, shredding the fat tissue raw material, and rinsing (eg, with physiological saline), thereby obtaining a rinsed fat tissue;
(2) centrifuging the rinsed fat tissue to obtain a layered mixture;
(3) discharging the excess liquid at the bottom and the grease on top from the layered mixture and collecting the intermediate layer (that is, the fat layer containing fat cells);
(4) subjecting the intermediate layer to mechanical emulsification to obtain a mechanically emulsified fat mixture (also called nano fat);
(5) centrifuging the mechanically emulsified fat mixture (may be combined or not) obtain a transparent (or substantially transparent) intermediate liquid layer, which is a fat primary extract; and
(6) subjecting the primary fat extract to filtration and sterilization to obtain the fat extract without added ingredients.

In another preferred embodiment, before the centrifugation step of step (5), the method further comprises subjecting the mechanically emulsified fat mixture to a freeze-thaw treatment.

In another preferred embodiment, in step (5), the mechanically emulsified fat mixture is freeze-thawed one or more times (eg, 1, 2, 3, 4, 5 times), and then the thawed fat mixture is centrifuged, and the clear liquid in the middle of the centrifuge tube is collected, which is the fat primary extract.

In another preferred embodiment, in step (6), the filtration and sterilization are performed through a filter (eg, a 0.22 µm filter).

In another preferred embodiment, in step (6), the filtration and sterilization are performed by first passing through a first filter that can filter out cells, and then passing through a second filter(such as a 0.22 µm filter) that can filter out pathogens (such as bacteria).

In another preferred embodiment, in step (6), further comprising sub-packaging the fat extract to form a sub-packed product. (The sub-packaged extract may be stored at -20 °C for later use; may be thawed at room temperature then used directly, or thawed and stored at a low temperature (eg, 4°C) for a period of time before use).

In another preferred embodiment, step (a) of the method further comprises providing a fat tissue, and centrifuging the fat tissue (preferably at 800-2500 rpm, more preferably at 1000-1500 rpm, most preferably at 1200 rpm) to obtain an intermediate fat layer located in the middle layer.

In another preferred embodiment, step (b) of the method further comprises freezing and thawing (preferably repeated freezing and thawing, such as repeated freezing and thawing 1-3 times) to obtain nano fat.

In another preferred embodiment, the emulsification is mechanical emulsification.

In another preferred embodiment, the emulsification is mechanical emulsification by repeatedly blowing through a syringe for many times (eg, 30-200 times, preferably 50-150 times).

In another preferred embodiment, the emulsification is a method of breaking up by a tissue homogenizer.

In the third aspect of the present invention, it provides a pharmaceutical or cosmetic composition, comprising (a) the fat extract of the present invention; and/or (b) a pharmaceutically or cosmetically acceptable carrier or excipient.

In another preferred embodiment, the cosmetically acceptable carrier or excipient is selected from the group consisting of moisturizing agent, antioxidant, anti-ultraviolet agent, preservative, film-forming agent, oil-soluble gelling agent, organic modified clay mineral, resin, antibacterial agent, fragrance, salt, pH adjuster, chelating agent, cooling agent, anti-inflammatory agent, skin beautifying ingredient, vitamin, amino acid, nucleic acid, hormone, inclusion compound, and a combination thereof.

In another preferred embodiment, the pharmaceutical composition comprises powder, granule, capsule, injection, tincture, oral liquid, tablet or lozenge.

In another preferred embodiment, the formulation of the cosmetic composition is a solid formulation, a semi-solid formulation, or a liquid formulation, such as solution, gel, cream, lotion, ointment, cream, paste, cake, powder, patch, etc.

In another preferred embodiment, the pharmaceutical or cosmetic composition is used for anti-aging, promoting proliferation of human skin fibroblasts, promoting skin fibroblast collagen synthesis, repairing after sun exposure, repairing skin barrier, repairing UV-induced DNA damage, lightening spots, anti-dark circles, and a combination thereof.

In another preferred embodiment, the pharmaceutical or cosmetic composition is used for resisting skin photoaging, promoting skin rejuvenation.

In another preferred embodiment, the cosmetic composition is used for skin care and beauty.

In another preferred embodiment, in the cosmetic composition, the mass percentage of the fat extract is 5wt%, preferably 1-20wt%, based on the total weight of the cosmetic composition.

In another preferred embodiment, the pharmaceutical or cosmetic composition further comprises additional component selected from the group consisting of whitening or freckle removing component, anti-inflammatory component, antioxidant component, anti-ultraviolet component, and a combination thereof.

In another preferred embodiment, the pharmaceutical composition comprises powder, granule, capsule, injection, tincture, oral liquid, tablet or lozenge.

In another preferred embodiment, the formulation of the cosmetic composition is a solid formulation, a semi-solid formulation, or a liquid formulation, such as solution, gel, cream, lotion, ointment, cream, paste, cake, powder, patch, etc.

In the fourth aspect of the present invention, it provides a method for preparing a pharmaceutical or cosmetic composition, comprising the steps of: mixing the fat extract herein with a pharmaceutically or cosmetically acceptable carrier to form a pharmaceutical or cosmetic composition.

In the fifth aspect of the present invention, it provides a skin care method, comprising the step of: administering the fat extract of the present invention to an individual in need thereof.

In another preferred embodiment, the method can be used in combination with the methods of moisturizing, anti-inflammatory, repairing after sun exposure, repairing skin barrier, repairing UV-induced DNA damage, whitening, lightening spots, anti-glycation and the like.

In the sixth aspect of the present invention, it provides a non-therapeutic method for culturing fibroblasts *in vitro,* comprising the steps of:
(i) providing a fat extract without added ingredients obtained by the method described in the second aspect of the present invention;
(ii) culturing fibroblasts in the presence of the fat extract to promote the proliferation of fibroblasts, promote the anti-aging of fibroblasts, and/or promote production of type I collagen in fibroblasts.

In the seventh aspect of the present invention, it provides a method for culturing fibroblasts *in vitro,* comprising the steps of:
(i) providing a fat extract without added ingredients obtained by the method described in the second aspect of the present invention;
(ii) adding the fat extract without added ingredients obtained in step (i) to a fibroblast medium;
(iii) obtaining fibroblasts with enhanced viability, anti-aging, and/or enhanced collagen I production capacity.

In the eighth aspect of the present invention, it provides a method for (a) promoting the proliferation of fibroblasts; (b) promoting the anti-aging of fibroblasts; and/or (c) promoting the production of type I collagen in fibroblasts, the method comprises the steps of: administering the fat extract without added ingredients of the present invention to a subject in need thereof.

In another preferred embodiment, the fat extract without added ingredients is obtained by the method described in the second aspect of the present invention;

In another preferred embodiment, the subject is human or non-human mammal.

In another preferred embodiment, the non-human mammal is cat, dog, pig, cow, sheep or monkey.

It should be understood that, within the scope of the present invention, the above technical features of the present invention and the technical features specifically described in the following descriptions (such as the examples) can be combined with each other to form a new or preferred technical solution. Due to space limitations, they will not be repeated herein.

### DESCRIPTION OF THE DRAWINGS

Figure 1 shows that different concentrations of fat extract liquid promote skin fibroblast proliferation *in vitro,* (A) cell morphology; (B) statistical results of cell proliferation.
Figure 2 shows that different concentrations of fat extract liquid improve the anti-photoaging ability of skin fibroblasts *in vitro,* (A) cell morphology; (B) statistical results of cell survival.
Figure 3 shows that different concentrations of fat extract liquid improve the anti-photoaging ability of skin fibroblasts *in vitro,* (A) cell ROS staining; (B) intracellular ROS flow cytometry analysis results.
Figure 4 shows that different concentrations of fat extract liquid resist the photoaging of skin fibroblasts, (A) cell beta-gal staining; (B) statistical analysis results of cell beta-gal staining; (C) cell phalloidin staining.
Figure 5 shows that different concentrations of fat extract promote the expression of type I collagen in skin fibroblasts.

In each figure, "control" represents a control, and "FE" represents the fat extract of the present invention.

### DETAILED DESCRIPTION OF THE INVENTION

After extensive and in-depth research, the present inventor has developed a fat extract without added ingredients for the first time. In the present invention, after the fat tissue is subjected to a series of treatments such as mechanical cutting, chylosis, repeated freezing and thawing, etc., the fat extract without oil droplets and living cell components is further extracted by the method of centrifugation. It was confirmed by *in vitro* cell experiments that fat extract can inhibit oxidative stress in skin fibroblasts, improve the anti-apoptotic ability of skin fibroblasts, and promote cell proliferation and collagen synthesis, suggesting that it has the effect of resisting oxidative damage to the skin and promoting skin rejuvenation. The present invention has been completed on this basis.

### Terms

Unless otherwise defined, all technical and scientific terms used herein have the same meaning as commonly understood by one of ordinary skill in the art to which this invention belongs.

As used herein, when used in reference to a specifically recited value, the term "about" means that the value may vary by no more than 1% from the recited value. For example, as used herein, the expression "about 100" includes all values between 99 and 101 (eg, 99.1, 99.2, 99.3, 99.4, etc.).

As used herein, the terms "contain" or "comprise (include)" may be open form, semi-closed form, and closed form. In other words, the terms also include "substantially consisting of' or "consisting of".

As used herein, "IGF-1" refers to insulin-like growth factors-1.

As used herein, "BDNF" refers to brain-derived neurotrophic factor.

As used herein, "GDNF" refers to glial cellline-derived neurotrophic factor.

As used herein, "bFGF" refers to basic fibroblast growth factor.

As used herein, "VEGF" refers to vascular endothelial growth factor.

As used herein, "TGF-β1" refers to transforming growth factor-β1.

As used herein, "HGF" refers to hepatocyte growth factor.

As used herein, "PDGF" refers to platelet derived growth factor.

As used herein, "EGF" refers to epidermal growth factor.

As used herein, "NT-3" refers to neurotrophins-3.

As used herein, "GH" refers to growth hormone.

As used herein, "G-CSF" refers to granulocyte colony stimulating factor.

### Fat Extract

As used herein, the term of "extract of the present invention", "fat extract of the present invention", "fat extract without added ingredients of the present invention ", "fat extract without additives of the present invention", etc. are used interchangeably, refer to the adipose tissue-derived extract (or extract liquid) prepared without adding any solution, solvent, small molecule, chemical, and biological additive during the preparation of the fat extract (except for the rinsing step). A typical method for preparing the extract of the present invention is described as in the second aspect of the present invention. In addition, it should be understood that although it is not necessary to add any additives (or added ingredients) during the preparation process of the extracts of the present invention, some or small amounts of safe substances (such as small amount of water) that do not negatively or adversely affect the activity of the extract herein may also be added.

Although the content of some cytokines in the extract of the present invention is relatively low, due to the coexistence of a variety of different natural factors, the extract of the present invention can act synergistically, so as to play a safe and efficient effect, especially for fibroblasts, has multiple distinct functions of promoting the proliferation of fibroblasts, promoting the anti-aging of fibroblasts, and promoting the production of type I collagen in fibroblasts.

### Oxidative stress and skin damage

Oxidative stress (OS) means that the reactive oxygen species (ROS) increases *in vivo* and participates in the formation of oxidative biomacromolecules when ROS is excessive produced or metabolic disorders occur, and exceeds the scavenging ability of the endogenous antioxidant defense system under the action of harmful stimuli, thereby directly or indirectly oxidizing or damaging DNA, proteins, and lipids, ultimately leading to oxidative damage to cells. On the one hand, skin is an integral part of the body system, and the oxidative stress caused by the imbalance of the system environment may affect the skin; on the other hand, as a defense barrier between the human body and the outside world, skin can be more and more directly exposed to oxidative stress caused by various external stimuli (especially UV rays in sunlight) compared with other organs of the body, thereby causing various skin problems.

Keratinocytes and fibroblasts are important cellular components of the skin epidermis and dermis, respectively, and fibroblasts are mainly involved in the synthesis of collagen and elastic fibers. These two types of cells are also the target sites of middle-wave ultraviolet (UVB). In the process of skin defense against external stimuli, the intracellular inflammatory signaling mechanism is activated and participates in the body's immune and inflammatory responses by expressing or secreting a series of inflammatory cytokines. In addition, UV may also cause apoptosis of two kinds of cells, and the possible mechanisms include direct damage to DNA, induction of ROS formation, and activation of cell membrane surface death receptors.

### USE

As used herein, the term "pharmaceutical or cosmetic composition" comprises (a) the fat extract of the present invention; and (b) a pharmaceutically or cosmetically acceptable carrier or excipient. In addition, the pharmaceutical composition also comprises health care product composition, and the cosmetic composition comprises skin care product.

The fat extract of the present invention may be prepared into a pharmaceutical composition, which may be a formulation such as tablets, capsules, powders, granules, solutions, lozenges, jellies, cream preparations, syrups, suspensions, tinctures, mud dressings, liniment, lotions, aerosols, and the like. The medicine can be prepared by generally known preparation techniques, and suitable pharmaceutical additives can be added into the medicine.

Examples of pharmaceutical additives include excipients, binders, decomposers, lubricants, flow aids, suspending agents, emulsifiers, stabilizers, moisturizers (wetting agents), preservatives, solvents, solubilizers, preservatives, flavoring agent, sweeteners, dyes, fragrances, propellants, etc. These pharmaceutical additives can be selected and added in an appropriate amount within a range that does not affect the effects of the present invention.

The fat extract of the present invention may be prepared into a cosmetic composition, which can be a formulation such as emulsion, liquid, ointment, cream, paste, cake, powder, and the like.

To the extent that the effects of the present invention are not hindered, other ingredients commonly used in cosmetics may be added into the cosmetics of the present invention, such as film formers, oil-soluble gelling agents, organically modified clay minerals, resins, moisturizers, preservatives, antibacterial agents, flavors, salts, antioxidants, pH adjusters, chelating agents, cooling agents, anti-inflammatory agents, ingredients for skin beautification (whitening agents, cytoactive agents, skin roughness improving agents, blood circulation promoters, skin firming agents, anti-lipid leakage agents, etc.), vitamins, amino acids, nucleic acids, hormones, inclusion compounds, etc.

The oil-soluble gelling agent is selected from metal soaps such as aluminum stearate, magnesium stearate, and zinc myristate; amino acid derivatives such as N-lauroyl-L-glutamic acid, α,γ-di-n-butylamine; cyclodextrin fatty acid esters such as cyclodextrin palmitate, cyclodextrin stearate, and cyclodextrin 2-ethylhexanoic acid palmitate; sucrose fatty acid esters such as sucrose palmitate and sucrose stearate; benzylidene derivatives of sorbitol such as monobenzylidene sorbitol and dibenzylidene sorbitol; gelling agent of organically modified clay minerals such as dimethylbenzyldodecylammonium montmorillonite clay and dimethyldioctadecylammonium montmorillonite clay. One, two or more types of agents may be used as required.

Humectant includes glycerin, sorbitol, propylene glycol, dipropylene glycol, 1,3-butanediol, glucose, xylitol, maltitol, polyethylene glycol, hyaluronic acid, chondroitin sulfate, pyrrolidone carboxylate , polyoxyethylene methyl glucoside, polyoxypropylene methyl glucoside, etc.

Antibacterial preservative includes alkyl p-hydroxybenzoate, benzoic acid, sodium benzoate, sorbic acid, potassium sorbate, phenoxyethanol, etc.. Antibacterial agents include benzoic acid, salicylic acid, carbolic acid, sorbic acid, alkyl p-hydroxybenzoate, p-chloro-m-cresol, hexachlorophenol, benzalkonium chloride, chlorhexidine chloride, trichloro-N-carbanilide, triclosan, photosensitizer, phenoxyethanol, etc.

Antioxidant includes tocopherol, butylhydroxyanisole, dibutylhydroxytoluene, phytic acid, etc. PH regulators include lactic acid, citric acid, glycolic acid, succinic acid, tartaric acid, dl-malic acid, potassium carbonate, sodium bicarbonate, ammonium bicarbonate, etc, chelating agents include alanine, sodium ethylenediaminetetraacetate, sodium polyphosphate, sodium metaphosphate, phosphoric acid, etc, cooling agents include L-menthol, camphor, etc, anti-inflammatory agents include allantoin, glycyrrhetinic acid, glycyrrhizic acid, tranexamic acid, azulene, etc.

Ingredients for skin beautification include whitening agents such as placenta extract, arbutin, glutathione and saxifrage extract; cytoactive agents such as royal jelly, photoreceptor, cholesterol derivatives, calf blood extract; skin roughness improving agents; blood circulation promoters such as valeramide pelargonate, benzyl nicotinate, β-butoxyethyl nicotinate, capsaicin, gingerone, cantharidin tincture, ichthyol, caffeine, tannic acid, α-borneol, tocopherol nicotinate , inositol hexanicotinate, cyclomandelate, cinnarizine, tolazoline, acetylcholine, verapamil, stephane and γ-oryzanol; skin firming agents such as zinc oxide, tannic acid; anti-lipid leakage agents such as sulfur, vitamins include vitamin A such as vitamin A oil, rosin oil, rosin acetate, rosin palmitate; vitamin B2 such as riboflavin, riboflavin butyrate and flavin adenine nucleotides; vitamin B6 such as pyridoxine hydrochloride, pyridoxine dicaprylate, pyridoxine tripalmitate, vitamin B such as vitamin B12 and its derivatives, vitamin B15 and its derivatives; vitamin C such as L-ascorbic acid, L-ascorbyl dipalmitate, sodium L-ascorbate-2-sulfate and dipotassium L-ascorbate phosphate diester; vitamin D such as ergocalciferol and cholecalciferol; vitamin E such as α-tocopherol, β-tocopherol, γ-tocopherol, dl-α-tocopherol acetate, dl-α-tocopherol nicotinate, dl-α-tocopherol succinate; vitamin H; vitamin P; niacin such as nicotinic acid, benzyl nicotinate, niacinamide; pantothenic acid such as calcium pantothenate, D-panthenol, pantothen ethyl ether and acetyl pantothen ethyl ether; biotin and the like.

Amino acids include glycine, valine, leucine, isoleucine, serine, threonine, phenylalanine, arginine, lysine, aspartic acid, glutamic acid, cystine, cysteine, methionine and tryptophan, nucleic acids include deoxyribonucleic acid and the like, and hormones include estradiol, vinyl estradiol and the like.

Preferred examples of the cosmetics of the present invention include skin care cosmetics, make-up cosmetics, and anti-ultraviolet cosmetics. For example, the basic cosmetics such as lotions, creams, lotions, sunscreens, mask materials, facial cleansers, and essences; and make-up cosmetics such as foundations, white powders, and blushes.

There is no particular limitation on the form of the product, and it may be liquid, emulsion, cream, solid, paste, gel, powder, multilayer, mousse, spray, and the like.

### The main advantages of the present invention include:

1. The fat extract of the present invention can effectively inhibit the oxidative stress in skin fibroblasts, at the same time improve the anti-apoptotic ability of skin fibroblasts, improve the resistance of cells to harsh environments, and save damaged skin conditions.
2. The fat extract of the present invention can synergistically, efficiently and balancedly promote cell proliferation and collagen synthesis, and promote skin rejuvenation.
3. The lipid droplets and cell components are removed from the fat extract of the present invention, thereby improving safety of using the extract of the present invention in a living body. Compared with obtaining nano-fat containing lipid droplets, vascular matrix components (SVF) and growth factors, the extract of the present invention contains no cell, so it does not belong to SVF, and has higher safety in use.
4. Unlike nano-fat which mainly works through SVF containing endothelial cells, adipose stem cells, macrophages and other cells, the extract of the present invention does not exert its effect through cells in SVF, but directly exerts its effect through natural factors.
5. The fat extract of the present invention can be derived from the applicator himself. In the future, the application of the allogeneic fat extract can be realized, and mass production and quality control can be realized.
6. Compared with the nano fat containing SVF, the fat extract of the present invention has cryopreservation convenience, operation simplicity and strong practicability.

The present invention will be further explained below in conjunction with specific embodiments. It should be understood that these embodiments are only used to illustrate the present invention and not to limit the scope of the present invention. The experimental methods that do not indicate specific conditions in the following examples are generally performed under the conventional conditions, such as the conditions described in Sambrook et al., Molecular Cloning: Conditions Described in Laboratory Manual (New York: Cold Spring Harbor Laboratory Press, 1989), or according to the manufacturer's instructions. Unless indicated otherwise, percentage and parts are calculated by weight.

### Example 1. Preparation of fat extract

Fat was obtained from volunteers with informed consent. The method of fat extract was as follows:
(1) The fat was obtained by suction or surgical excision, cut into pieces, and rinsed three times with normal saline.
(2) The rinsed adipose tissue was put into a 50 ml centrifuge tube (about 30-50ml per tube), then put in a centrifuge and centrifuged at 1200 rpm for 3 minutes to obtain a layered mixture.
(3) The excess liquid at the bottom and grease on top were drained from the layered mixture, and the intermediate layer (ie, the fat layer containing adipocytes) was collected.
(4) The intermediate layer was blown about 60-120 times by two 10ml injection syringes connected with a tee tube to perform mechanical emulsification, thereby obtaining a mechanically emulsified fat mixture (also called nano fat).
(5) The mechanically emulsified fat mixture (may be combined or not) was put into a 50ml test tube, centrifuged at 1500rpm for 5 minutes, and the transparent liquid in the middle of test tube was collected, which is a fat primary extract. Alternatively, the mechanically emulsified fat mixture was placed in a -80 °C refrigerator (or liquid nitrogen) for freezing, and then thawed in a water bath (eg, placed in a water bath at 20-37°C), and freeze-thaw was repeated 1-2 times. The thawed mixture was centrifuged at 1500 rpm for 5 minutes, and the transparent liquid in the middle of the centrifuge tube was collected, which is the fat primary extract.
(6) The fat primary extract was passed through a 0.22 µm filter to sterilize and remove live cells that may be mixed, thereby obtaining a fat extract without added ingredients. After the fat extract was sub-packaged, it was stored at -20 °C until use. It can be used directly after thawing at room temperature, or stored at a low temperature (eg, 4°C) for a period of time after thawing then use.

For the prepared cell-free fat extract, ELISA immunosorbent assay kit was used to detect the content of cytokines, including IGF-1, BDNF, GDNF, bFGF, VEGF, TGF-β1, HGF, PDGF and other cytokines. The average concentrations of 6 samples detected are as follows: IGF-1 (9840.6 pg/ml), BDNF (1764.5 pg/ml), GDNF (1831.9 pg/ml), bFGF (242.3 pg/ml), VEGF (202.9 pg/ml), TGF-β1 (954.5 pg/ml), HGF (898.4 pg/ml), and PDGF (179.9 pg/ml).

### Example 2. The effect of fat extract on the proliferation of human skin fibroblasts

Human skin fibroblasts were isolated from neonatal foreskin tissue, and then inoculated in high-glucose DMEM medium containing 10% fetal bovine serum. The cells were passaged once every 3 days, and the third and fourth passages were used in the experiment.

Human skin fibroblasts were seeded in 96-well plates at a density of 1000 cells per well, and different concentrations (1%-5%) of fat extract were added respectively, and 6 sub-wells were set for each concentration. After the cells were incubated for 72 hours, cell proliferation was detected by CCK8 kit and OD value was determined. The experiment was repeated three times to obtain the mean and standard deviation.

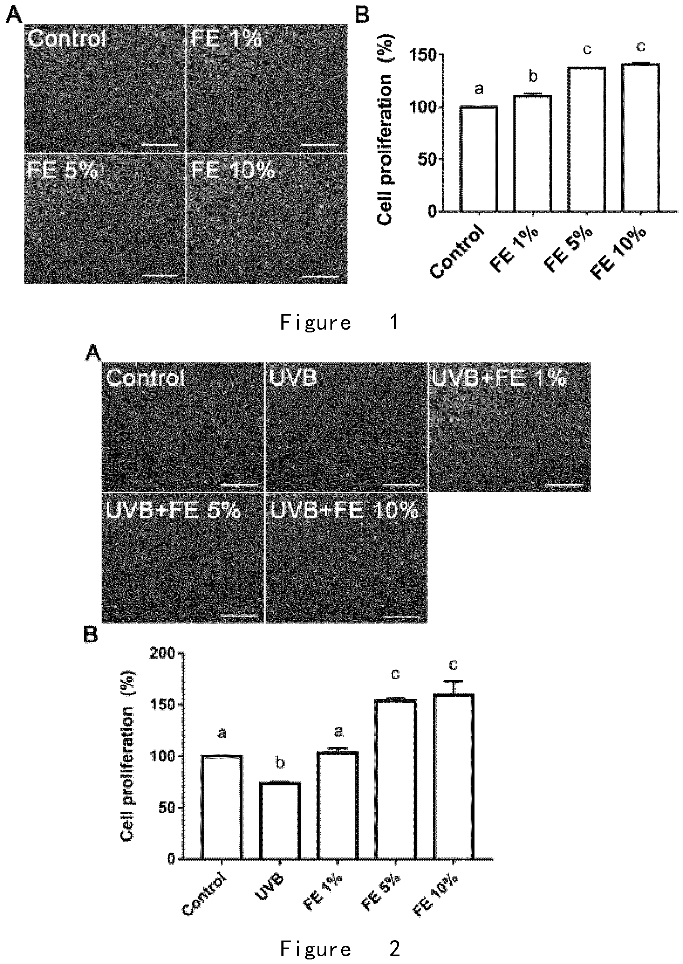

Result:
Human skin fibroblasts were cultured with different concentrations of fat extract. CCK-8 detection showed that the number of cells in the extract-added group increased significantly compared with the control group after 72 hours of culture and the number of cells was in a dose-dependent manner (Figure 1).

Human skin fibroblasts were cultured with different concentrations of fat extracts and subjected to ultraviolet irradiation. CCK-8 detection showed that the number of cells decreased after ultraviolet irradiation after 72 hours of culture, and the number of cells in the extract-added group was significantly increased compared with the irradiation group (Figure 2).

### Example 3. The effect of fat extract on skin tissue anti-aging and oxidative stress

Skin fibroblasts were treated with different concentrations (1%-5%) of fat extracts for 24 hours, and the content of intracellular reactive oxygen species (ROS) was detected by flow cytometry after ultraviolet irradiation (100 mJ/cm²).

48 hours after irradiation, the cell cycle was detected by flow cytometry.

72 hours after irradiation, cell proliferation was detected by CCK8 kit, and senescent cells were stained with beta-gal and phalloidin to detect the degree of aging. In addition, the RNA of cells was extracted, and the expression of type I collagen was detected by RT-PCR.

### Result:

### 3.1 ROS content

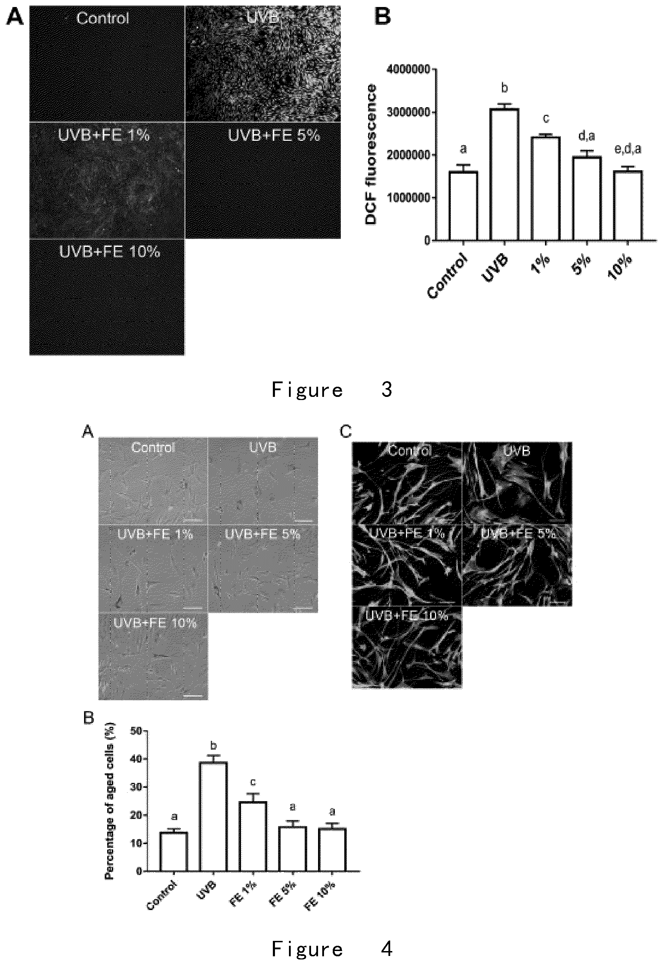

Human skin fibroblasts were cultured with different concentrations of fat extract, and intracellular ROS staining was performed immediately after UV irradiation. The accumulation of intracellular ROS after UV irradiation was observed. In the extract-added group, the intracellular ROS content decreased significantly (Figure 3).

### 3.2 Aging degree

Human skin fibroblasts were cultured, added with different concentrations of fat extract, irradiated with ultraviolet light and then cultured for 72 hours Beta-gal and phalloidin staining were performed. The results showed that the number of beta-gal positive senescent cells in the UV-irradiated group was increased and the number of senescent cells in the extract-treated group was significantly reduced.

Phalloidin staining showed that under ultraviolet irradiation, the morphology of fibroblasts was in a spread state in the control group, indicating aging; while in the extract-treated group, fibroblasts maintained a long spindle shape, indicating the resistance against aging caused by ultraviolet radiation (Figure 4).

This shows that the fat extract without added ingredients of the present invention can effectively resist the aging process of fibroblasts (including aging caused by environmental factors such as ultraviolet radiation).

### 3.3 Synthesis of Type I Collagen

Human skin fibroblasts were cultured, added with different concentrations of fat extract irradiated with ultraviolet light and then cultured for 72 hours. The cells were collected to extract RNA and total protein, and the expression of type I collagen was detected by RT-PCR.

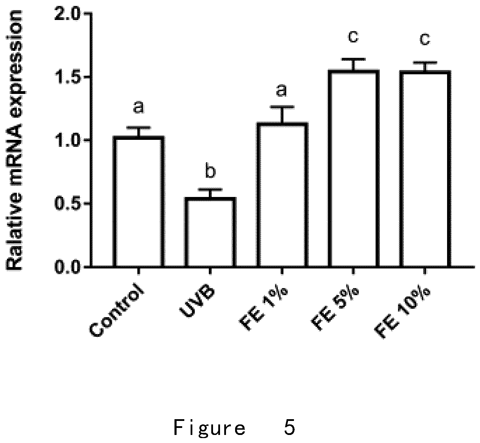

The results showed that the expression of type I collagen by cells was decreased in the ultraviolet radiation group (UVB group), while the expression of type I collagen was increased in the extract-treated group, unexpectedly even higher than that in the non-irradiated group (control group) (Figure 5).

### Discussion

Tonnard first proposed the concept of nano-fat. Nano-fat containing lipid droplets, vascular matrix components (SVF) and growth factors can be obtained after mechanical emulsification of the fat obtained by liposuction. Nano-fat mainly works through SVF. SVF contains endothelial cells, adipose stem cells, macrophages, etc. On the one hand, cells can directly participate in tissue formation, and on the other hand, cells can promote tissue regeneration by secreting cytokines.

Nano-fat is a product obtained by chylosis after mechanical cutting of adipose tissue, which contains lipid droplets, living stromal cells and various growth factors, and is used in soft tissue filling. Previous studies have found that subcutaneous injection of nano-fat in photoaging nude mice can promote new blood vessel formation. Because nano-fat contains stromal cells and growth factors, previous studies have believed that the living cell components in nano-fat play an important role, but it is still unclear whether the cellular components are necessary in anti-skin photoaging. The present invention unexpectedly found that, through *in vitro* cell experiments, it was confirmed that the fat extract without oil droplets and living cell components can inhibit oxidative stress in skin fibroblasts, improve the anti-apoptotic ability of skin fibroblasts, and promote cell proliferation and collagen synthesis, suggesting that it has the effect of resisting oxidative damage to the skin and promoting skin rejuvenation.

The difference from the previous study is that the living cells and lipid droplets in the nano-fat are removed by centrifugation and filtration in the present invention, and the growth factors were retained. *In vitro* cell photoaging model confirms that the fat extract can improve the anti-apoptotic ability of skin fibroblasts, promote cell proliferation and collagen synthesis, suggesting that it has the application prospect of resisting skin photoaging and promoting skin rejuvenation.

Compared with SVF-containing nano-fat, the fat extract of the preaent invention has wider application prospects and has significant advantages: for example, firstly, lipid droplet components are removed, and possible side effects are reduced; secondly, the cell components are removed, thus removing the immunogenicity, the application of allogeneic fat extract can be realized in the future, and mass production and quality control can be realized; thirdly, it is easy to cryopreserve and maintain biological activity, and no protective agent is required during cryopreservation, avoiding the contamination of other chemical components.

All the documents cited herein are incorporated into the invention as reference, as if each of them is individually incorporated. Further, it would be appreciated that, in light of the above-described teaching of the invention, the skilled in the art could make various changes or modifications to the invention, and these equivalents are still in the scope of the invention defined by the appended claims of the application.

## Claims

1. Use of a fat extract without added ingredients, for the manufacture of a composition or product, the composition or product is used for one or more uses selected from the group consisting of (a) promoting proliferation of fibroblasts; (b) promoting anti-aging of fibroblasts; (c) promoting production of type I collagen in fibroblasts.

2. The use according to claim 1, wherein the aging comprises cell aging caused by ultraviolet irradiation.

3. The use according to claim 1, wherein the composition or product is also used for preventing and/or repairing skin damage caused by damage or decreased vitality of fibroblasts.

4. The use according to claim 1, wherein the skin damage comprises photoaging, polymorphic light eruption.

5. The use according to claim 1, wherein the composition is also used for preventing and/or treating skin disease.

6. The use according to claim 1, wherein the fat extract contains no cell and no lipid droplet.

7. The use according to claim 1, wherein the "without added ingredients" refers to no solution, solvent, small molecule, chemical, and biological additive are added during the preparation of the fat extract except rinsing step.

8. The use according to claim 1, wherein the cell-free fat extract contains one or more components selected from the group consisting of IGF-1, BDNF, GDNF, HGF, bFGF, VEGF, TGF-β1, HGF, PDGF, EGF, NT-3, GH, G-CSF, and a combination thereof.

9. The use according to claim 8, wherein the fat extract without added ingredients comprises one or more features selected from the group consisting of:
in the fat extract without added ingredients, the concentration of IGF-1 is 5000-30000 pg/ml, preferably 6000-20000 pg/ml, more preferably 7000-15000 pg/ml, more preferably 8000-12000 pg/ml, more preferably 9000-11000 pg/ml, more preferably 9500-10500 pg/ml;
in the fat extract without added ingredients, the concentration of BDNF is 800-5000 pg/ml, preferably 1000-4000 pg/ml, more preferably 1200-2500 pg/ml, more preferably 1400-2000 pg/ml, more preferably 1600-2000 pg/ml, more preferably 1700-1850 pg/ml;
in the fat extract without added ingredients, the concentration of GDNF is 800-5000 pg/ml, preferably 1000-4000 pg/ml, more preferably 1200-2500 pg/ml, more preferably 1400-2000 pg/ml, more preferably 1600-2000 pg/ml, more preferably 1700-1900 pg/ml;
in the fat extract without added ingredients, the concentration of bFGF is 50-600 pg/ml, preferably 100-500 pg/ml, more preferably 120-400 pg/ml, more preferably 150-300 pg/ml, more preferably 200-280 pg/ml, more preferably 220-260 pg/ml;
in the fat extract without added ingredients, the concentration of the VEGF is 50-500 pg/ml, preferably 100-400 pg/ml, more preferably 120-300 pg/ml, more preferably 150-250 pg/ml, more preferably 170-230 pg/ml, more preferably 190-210 pg/ml;
in the fat extract without added ingredients, the concentration of TGF-β1 is 200-3000 pg/ml, preferably 400-2000 pg/ml, more preferably 600-1500 pg/ml, more preferably 800-1200 pg/ml, more preferably 800-1100 pg/ml, more preferably 900-1000 pg/ml;
in the fat extract without added ingredients, the concentration of the HGF is 200-3000 pg/ml, preferably 400-2000 pg/ml, more preferably 600-1500 pg/ml, more preferably 600-1200 pg/ml, more preferably 800-1000 pg/ml, more preferably 850-950 pg/ml; and/or
in the fat extract without added ingredients, the concentration of PDGF is 50-600 pg/ml, preferably 80-400 pg/ml, more preferably 100-300 pg/ml, more preferably 140-220 pg/ml, more preferably 160-200 pg/ml, more preferably 170-190 pg/ml.

10. The use according to claim 8, wherein the fat extract without added ingredients comprises one or more features selected from the group consisting of:
the weight ratio of IGF-1 to VEGF is 20-100:1, preferably 30-70:1, more preferably 40-60:1, and most preferably 45-55:1;
the weight ratio of BDNF to VEGF is 2-20:1, preferably 4-15:1, more preferably 6-12:1, and most preferably 8-9.5:1;
the weight ratio of GDNF to VEGF is 2-20:1, preferably 4-15:1, more preferably 6-12:1, and most preferably 8.5-9.5:1;
the weight ratio of bFGF to VEGF is 0.2-8:1, preferably 0.5-5:1, more preferably 0.6-2:1, more preferably 0.8-1.6:1, and most preferably 1-1.5: 1;
the weight ratio of TGF-β1 to VEGF is 1-20:1, preferably 1-15:1, more preferably 1-10:1, more preferably 2-8:1, more preferably 4-6:1;
the weight ratio of HGF to VEGF is 1-20:1, preferably 1-15:1, more preferably 1-10:1, more preferably 2-8:1, more preferably 4-5.5:1; and/or
the weight ratio of PDGF to VEGF is 0.1-3:1, preferably 0.2-2:1, more preferably 0.4-1.5:1, and most preferably 0.7-1.2:1.

11. The use according to claim 1, wherein the fat extract without added ingredients is prepared by the following method, and the method comprises the following steps:
(1) providing a fat tissue raw material, shredding the fat tissue raw material, and rinsing, thereby obtaining a rinsed fat tissue;
(2) centrifuging the rinsed fat tissue to obtain a layered mixture;
(3) discharging the excess liquid at the bottom and the grease on top from the layered mixture, and collecting the intermediate layer;
(4) subjecting the intermediate layer to mechanical emulsification to obtain a mechanically emulsified fat mixture;
(5) centrifuging the mechanically emulsified fat mixture to obtain a transparent or substantially transparent intermediate liquid layer, which is a fat primary extract; and
(6) subjecting the fat primary extract to filtration and sterilization to obtain a fat extract without added ingredients.

12. A preparation method of the fat extract without added ingredients, comprises the following steps:
(1) providing a fat tissue raw material, shredding the fat tissue raw material, and rinsing, thereby obtaining a rinsed fat tissue;
(2) centrifuging the rinsed fat tissue to obtain a layered mixture;
(3) discharging the excess liquid at the bottom and the grease on top from the layered mixture, and collecting the intermediate layer;
(4) subjecting the intermediate layer to mechanical emulsification to obtain a mechanically emulsified fat mixture;
(5) centrifuging the mechanically emulsified fat mixture to obtain a transparent or substantially transparent intermediate liquid layer, which is a fat primary extract; and
(6) subjecting the fat primary extract to filtration and sterilization to obtain a fat extract without added ingredients.

13. A pharmaceutical or cosmetic composition, comprising (a) a fat extract prepared by the method of claim 12; and/or (b) a pharmaceutically or cosmetically acceptable carrier or excipient.

14. A method for preparing a pharmaceutical or cosmetic composition, comprising the steps of: mixing the fat extract prepared by the method according to claim 12 with a pharmaceutically or cosmetically acceptable carrier to form a pharmaceutical or cosmetic composition.

15. A non-therapeutic method for culturing fibroblasts *in vitro,* wherein the method comprises the steps of:
(i) providing a fat extract without added ingredients obtained by the method according to claim 12;
(ii) culturing fibroblasts in the presence of the fat extract to promote the proliferation of fibroblasts, promote the anti-aging of fibroblasts, and/or promote production of type I collagen in fibroblasts.
